# EUROPEAN PATENT APPLICATION

(11) **EP 4 226 911 A1**
(43) Date of publication of application: **16.08.2023**
(21) Application number: 22155916.4
(22) Date of filing: 09.02.2022
(51) Int. Cl.: A61K 8/60, A61Q 19/02, A61Q 19/04

(54) **ACTIVE AGENT MODULATING THE ACTIVITY OF AN ION CHANNEL FOR USE IN REGULATION OF SKIN PIGMENTATION**

(71) Applicant: CUTANEON - Skin & Hair Innovations GmbH, 22339 Hamburg (DE)
(72) Inventor: PAUS, Ralf, 22339 Hamburg (DE); BÍRÓ, Tamás, 4032 Debrecen (HU); MARDARYEV, Andrei, St Saviour, Jersey (GB)
(74) Representative: WSL Patentanwälte Partnerschaft mbB

(57) **Abstract**

In order to provide new active agents for actively controlling and/or treating skin pigmentation in subjects without undesired side effects the present invention discloses active agents activating, enhancing, inactivating, blocking or dampening the cellular response of the transient receptor potential ion channel TRPM5 or interfering with the expression of said ion channel.

## Description

The present invention is directed to an active agent for use in skin pigmentation regulation, particularly for use in the treatment of skin pigmentation conditions and disorders, wherein said active agent modulates the activity of an ion channel, including but not limited to activation, enhancement, inactivation or blocking an ion channel or dampening the cellular response induced by an ion channel or interfering with the expression of said ion channel.

Further, the present invention is directed to a composition for use as a cosmetic or medicament in the treatment of skin pigmentation said composition comprising at least one active agent that modulates the activity of an ion channel, including but not limited to activation, enhancement, inactivation or blocking an ion channel or dampening the cellular response induced by an ion channel or interfering with the expression of said ion channel.

In addition, the present invention is directed to a non-therapeutic method of skin pigmentation regulation, wherein an effective amount of at least one active agent that modulates the activity of an ion channel, including but not limited to activation, enhancement, inactivation or blocking an ion channel or dampening the cellular response induced by an ion channel or interfering with the expression of said ion channel, is administered to a subject.

Melanin is the skin's natural protection against ultraviolet radiation. It is the main determinant of skin and hair color and is produced and distributed in the skin through a process termed melanogenesis. Melanin is initially synthesized within intracellular organelles (melanosomes) in melanocytes and then distributed to neighboring keratinocytes. Strictly speaking, pigmentation encompasses not only melanin synthesis *per se* but several steps including transfer and processing of melanin (mostly towards and within epidermal keratinocytes) that underlie skin and hair color. Ultraviolet (UV) radiation exposure is strongly associated with skin pigmentation. UV radiation-induced DNA damage stimulates melanogenesis through the up-regulation and activation of tyrosinase, the enzyme that catalyzes the rate-limiting step of melanin synthesis.

Hyperpigmentation due to melanin overproduction is implicated in a variety of skin pigmentation conditions/disorders, including, but not limited to, lentigines (aging spots), solar lentigo (dark spots due to chronic sun exposure), melasma (larger dark patches), chloasma (skin discoloration caused by hormones), ephelides (freckles), (hyper)pigmented birthmarks such as nevus of Ota, Mongolian spots, cafe-au-lait spots, pigmented nevi (moles), hyperpigmentation as a result of skin damage (e.g. post-inflammatory hyperpigmentation, post-infection hyperpigmentation, post-blister hyperpigmentation, post-burn hyperpigmentation, post-traumatic hyperpigmentation, post-birth hyperpigmented stretch marks [striae distensae]), and dark spots of any kind. The treatment of these conditions/disorders, as well as of those individuals who claim to have "darker than desired" skin color (and hence intend to transform it to a lighter one), involve skin lightening products and technologies.

Currently, there are multiple actives on the market that are claimed to be instrumental for the treatment of hyperpigmentation. Several of said actives belong to the group of vitamins (e.g. vitamin C, alpha tocopherol or vitamin E, niacinamide). The disadvantages of said vitamins are that they do not specifically, or only marginally, target melanocytes and the process of melanogenesis, and their putative pigmentation-suppressing effects are claimed to be mainly attributed to their antioxidant actions on the skin at large.

In addition, certain phenolic compounds (e.g. hydroquinone and derivatives) were also claimed to be effective in the treatment of hyperpigmentation as they were shown to inhibit melanocyte tyrosinase, the key enzyme of melanogenesis. However, their widespread use is hampered by their extensive chronic adverse effects (e.g. contact dermatitis, sclera and nail hyperpigmentation, impaired wound healing) and there are severe safety concerns as hydroquinone was shown to cause DNA damage and hence may increase the risk of carcinogenesis.

On the other hand, generalized or localized skin melanin pigment suppression or depletion is a hallmark of a multitude of hypopigmentation skin conditions/disorders, including, but not limited to, vitiligo (a hypopigmentation condition characterized by patches of depigmentation throughout the skin), hypopigmentation as a result of skin damage (e.g. post-inflammatory hypopigmentation, post-infection hypopigmentation, post-blister hypopigmentation, post-burn hypopigmentation, post-traumatic hypopigmentation, post-birth hypopigmented stretch marks [striae distensae], post-bleaching hypopigmentation), and white or light spots of any kind. The treatment of these conditions, as well as of those individuals who claim to have "whiter or lighter than desired" skin color (and hence intend to transform it to a darker one), involve skin darkening and/or "UV-free tanning" products and technologies.

Despite not being life-threatening complications, the aforementioned skin pigmentation conditions/disorders may lead to severe psychological distress in affected individuals.

Besides the classical UV irradiation to induce the photoprotective tanning of the skin and the topically applied "artificial tanning" substances (e.g. dihydroxyacetone, melanins) that simulate natural pigmentation, the agents that have been most extensively tested are analoges of the melanocyte stimulating hormone (MSH). For example, alpha-MSH mimetic peptide, afamelanotide/melanotan, was shown to induce skin pigmentation in human. However, afamelanotide/melanotan have caused great concerns because it was found to trigger the development/eruption of melanocytic nevi and thereby increase the risk of developing malignant melanoma.

In light of the above, there is a continuous need for active agents for actively controlling and/or treating skin pigmentation in subjects without undesired side effects.

The inventors of the present invention found that the transient receptor potential ion channel TRPM5 is expressed in basal layers of the epidermis, where the melanin producing melanocytes are located.

"Ion channels" are pore-forming proteins located in biological membranes. Through the pores of said proteins ions may pass the membrane down their electrochemical gradient. By either opening or closing the pore ion channels are capable of gating and controlling the flow of ions across the membranes thereby modulating the intracellular concentrations of ions. Alteration of intracellular ion concentrations affect a plethora of cellular responses and processes including, but not limited to, e.g. growth, differentiation, survival, death, mediator release, immune mechanisms, etc.

"Transient receptor potential channels" (TRP channels) are a group of ion channels located mostly on the plasma membrane, and there are about 30 TRP channels including TRPC, TRPV, TRPM, TRPN, TRPA, TRPP and TRPML. Said ion channels have a relatively nonselective permeability to cations, including sodium, calcium and magnesium.

"TRPM5" is the official gene symbol for "transient receptor potential cation channel subfamily M member 5" and identifies the protein that is encoded by the TRPM5 gene in humans (NCBI Gene ID: 29850; HGNC: 14323; NCBI mRNA sequence: NM_014555.3; NCBI protein sequence: NP_055370.1; Status on June 7, 2020).

The inventors of the present application have found that the pheromone 2,5-dimethylpyrazine (in the following referred to as "DMP"), being an agonist of the transient receptor potential ion channel TRPM5, causing the channel to be open, promoted *ex vivo* human skin (epidermal) pigmentation. Indeed, when topically applied in human *ex vivo* skin organ cultures - a model that is suitable to assess multiple hallmarks of epidermal melanogenesis and melanin transfer -, DMP upregulated epidermal melanin content, the most important marker of melanogenesis, as verified by two independent histochemical technologies.

Further, as revealed by quantitative histomorphometry, topical DMP treatment also increased the level of the premelanosome protein, gp100 (also known as Pmel17 or Silver locus protein) which is known to be involved in melanosome-trafficking compartment, contributes to the functional integrity and assembly of melanosomes, and hence participates in *de novo* melanin synthesis. Moreover, topical DMP treatment also increased intraepidermal activity of tyrosinase, the rate-limiting enzyme of melanogenesis, as revealed by an *in situ* enzyme assay.

*Vice versa,* inhibition of TRPM5 using the selective inhibitor triphenylphosphine oxide (TPPO) consistently prevented the pigmentation-promoting effects of DMP.

In addition to the above active agents, the inventors identified a number of additional active agents acting on TRPM5 and, thus, being suitable for use in the treatment of skin pigmentation.

Concluding, the present invention is characterised by an active agent for use in the treatment of skin pigmentation, wherein said active agent activates, enhances, inactivates or blocks TRPM5 or dampens the cellular response induced by the transient receptor potential ion channel TRPM5 or interferes with the expression of said ion channel.

According alternatives of the present invention the active agent that activates, enhances, inactivates or blocks the ion channel or dampens the cellular response induced by the transient receptor potential ion channel TRPM5 is either an agonist or an antagonist of said ion channel.

As referred to herein an "agonist" of the transient receptor potential ion channel TRPM5 is a substance that binds to said ion channel and activates or enhances the ion channel thereby augmenting the cellular response linked to TRPM5.

As referred to herein an "antagonist" of the transient receptor potential ion channel TRPM5 is a substance that binds to said ion channel and blocks TRPM5 thereby dampening the cellular response to an agonist rather than activating or enhancing it like an agonist.

An "inverse agonist" of the transient receptor potential ion channel TRPM5 is a substance that binds to said ion channel and in turn induces an opposite the cellular response than seen when applying an agonist.

With respect to the transient receptor potential ion channel TRPM5 of the present invention the term "cellular response" is primarily to be understood as a change of ion concentration in the cell being the result of an agonist/antagonist/inverse agonist binding to the transient receptor potential ion channel TRPM5. However, the term also encompasses instances, where the binding of an agonist/antagonist/inverse agonist induces a cellular response without altering the intracellular ion concentration.

Accordingly an inventive agonist of the transient receptor potential ion channel TRPM5 activates or enhances said ion channel to produce the cellular response like an endogenous agonist does, whereas the inventive antagonist of said ion channel blocks or dampens the ion channel to produce the cellular response as usually activated/enhanced by an endogenous agonist, and the inventive inverse agonist modulates the ion channel to produce the opposite cellular response as usually activated/enhanced by endogenous agonists.

In those embodiments, where the active agent is an agonist of the transient receptor potential ion channel TRPM5, said active agent is used in the treatment of hypopigmentation conditions. In those embodiments, where the active agent is an antagonists/inverse agonists of the transient receptor potential ion channel TRPM5, said active agent is used in the treatment of hyperpigmentation conditions.

In specific embodiments of the invention the ion channel agonist/antagonist/inverse agonist used is a TRPM5 agonist selected from the group consisting of dimethylpyrazine, dimethylethylpyrazine, tetramethylpyrazine, 2-heptanone, eugenol, SID2848719 (CAS number 702636-90-6, SMILES, NC(=O)C1 (CCN(CC1)S(=O)(=O)c1ccc2OCCCOc2c1)N1CCCCC1), rutamarin, bergapten, xanthotoxin, isopimpinellin, carbachol, 3-deoxyglucosone, glucagon-like peptide 1, (E)-N-(3,4dimethoxybenzylidene)-2-naphthalene-1-yl)acetohydrazide, or a combination thereof.

In other embodiments of the invention the ion channel agonist/antagonist/inverse agonist used is a TRPM5 antagonist/inverse agonist selected from triphenylphosphine oxide, econazole, miconazole, chlorpromazine, or combinations thereof.

Even though the inventors are not aware of any such instance, it cannot be excluded, that there exist prior art skin pigmentation treatment compositions comprising one of the specific inventive active agents mentioned above as an ingredient, without attributing said ingredient any effect on TRPM5. In this event, such accidentially anticipated active agent shall be excluded from the scope of the invention by a corresponding disclaimer. Except for excluding said accidentially anticipated specific active agent said disclaimer shall not further affect the scope of the claims.

Non-limiting examples for active agents being accidentially anticipated in the prior art are
- chemical compounds being attributed no medical, pharmacological or biological activity at all by the corresponding prior art,
- chemical compounds being attributed another medical, pharmacological or biological activity by the corresponding prior art as compared to the herein disclosed specific inventive active agents, and/or
- chemical compounds being attributed the same medical, pharmacological or biological activity by the corresponding prior art as compared to the herein disclosed specific inventive active agents, but not being directed to TRPM5 but being directed to another target (e.g. receptor, enzyme, hormone, metabolite).

Alternatively, the TRPM5 agonist is an aptamer binding to the TRPM5 ion channel and activating or enhancing the ion channel to produce the cellular response, or the TRPM5 antagonist/inverse agonist is an aptamer binding to the TRPM5 ion channel and inactivating, blocking or dampening the ion channel to produce the cellular response.

The term "aptamer" as used herein refers to DNA, RNA or XNA oligonucleotide or peptide molecules that bind to a specific target molecule, such as ion channel molecules.

According to an alternative of the present invention, an active agent that interferes with the expression of the ion channel TRPM5 is either miRNA, siRNA or a ribozyme targeted to the TRPM5 gene or targeted to the mRNA corresponding to the TRPM5 gene.

The term "miRNA" refers to microRNA, i.e. small non-coding RNA molecules containing 21 to 23 nucleotides and functioning in RNA knock-down and post-transcriptional regulation of gene expression. miRNAs function via base-pairing with complementary sequences within mRNA molecules. As a result, these mRNA molecules are knocked-down, by cleavage, destabilization and/or less efficient translation of the mRNA.

The term "siRNA" refers to small interfering RNA, i.e. small non-coding RNA molecules, 20 to 25 base pairs in length. siRNA interferes with the expression of specific genes with complementary nucleotide sequences by degrading mRNA after transcription, thereby preventing translation.

According to the present invention a gene is "targeted" by a miRNA, siRNA or a ribozyme when the miRNA, siRNA or a ribozyme molecule selectively decreases or inhibits the expression of TRPM5. The phrase "selectively decrease or inhibit" as used herein refers to miRNA, siRNA or a ribozyme that affects the expression of TRPM5.

In specific embodiments of the invention miRNA or siRNA interfere with the gene expression of the ion channel TRPM5 by hybridizing under stringent conditions to the gene transcript, i.e. the TRPM5 mRNA, wherein hybridizing "under stringent conditions" means annealing to the target mRNA region, under standard conditions, e.g., high temperature (e.g. < 60 °C for 2 hours) and/or low salt content (e.g. 0.1xSSC) which tend to disfavor hybridization.

According to the present invention one of the above-mentioned active agents is used in the treatment of skin pigmentation. In specific embodiments of the invention the treatment is effected locally, i.e. in, on or at the skin area to be treated. In some embodiments of the invention said treatment is effected topically, wherein the term "topical" refers to a formulation that is applied to a particular place on the skin. In specific embodiments the topical application is epicutaneous, meaning that the agonist or antagonist is applied directly to the skin. In other embodiments of the invention the treatment is effected transdermally, wherein the term "transdermal" refers to a formulation that is applied across the Stratum corneum into the deeper skin layers, e.g. by injection with a standard needle or microneedles. In other embodiments of the invention the treatment is effected transappendageally, wherein the term "transappendageal" refers to an applied formulation that is permeating the skin via skin appendage structures (such as the hair follicles, sebaceous glands, and sweat glands) into the deeper skin layers.

The term "treatment" as used herein refers to any action resulting in the change of a physical condition. Particularly, the "treatment of skin pigmentation" refers to any change of an initial skin pigmentation condition, such as lentigines (aging spots), solar lentigo (dark spots due to chronic sun exposure), melasma (larger dark patches), chloasma (skin discoloration caused by hormones), ephelides (freckles), (hyper)pigmented birthmarks such as nevus of Ota, Mongolian spots, cafe-au-lait spots, pigmented nevi (moles), hyperpigmentation as a result of skin damage (e.g. post-inflammatory hyperpigmentation, post-infection hyperpigmentation, post-blister hyperpigmentation, post-burn hyperpigmentation, post-traumatic hyperpigmentation, post-birth hyperpigmented stretch marks [striae distensae]), dark spots of any kind, "darker than desired" skin color, vitiligo (a hypopigmentation condition characterized by patches of depigmentation throughout the skin), hypopigmentation as a result of skin damage (e.g. post-inflammatory hypopigmentation, post-infection hypopigmentation, post-blister hypopigmentation, post-burn hypopigmentation, post-traumatic hypopigmentation, post-birth hypopigmented stretch marks [striae distensae], post-bleaching hypopigmentation), white or light spots of any kind, and "whiter or lighter than desired" skin color. In other words, the present invention is directed to any kind of skin pigmentation regulation.

In specific embodiments of the invention the above-mentioned active agent is used as a cosmetic in the treatment of skin pigmentation. Particularly, the use as a cosmetic occurs non-therapeutically, but in order to achieve a change of an initial skin pigmentation condition, such as unwanted skin color (darker or lighter/whiter than desired), wherein said initial condition is not caused by a disease or disorder.

In those embodiments, where the above mentioned active agent is used as a cosmetic the active agent used should be cosmetically acceptable, wherein "cosmetically acceptable" means that the active agent should not be toxic or harmful or have any other detrimental side effects upon application on skin.

In other specific embodiments of the invention the above mentioned active agent is used as a medicament in the topical treatment of skin pigmentation disorder, wherein the term "disorder" refers to any functional abnormality or disturbance of the normal healthy condition, and the term "medicament" refers to a substance useful in curing, treating or preventing a condition of disorder.

In those embodiments, where the above mentioned active agent is used as a medicament the active agent used should be pharmaceutically acceptable, wherein "pharmaceutically acceptable" means that the active agent should not be toxic or harmful or have any other detrimental side effects upon application on skin.

In some embodiments at least one of the inventive active is used as an ingredient of a composition for use as a cosmetic or medicament in the topical treatment of skin pigmentation said composition further comprising at least one auxiliary agent selected from the group consisting of carriers, excipients, adjuvants, diluents, and disintegrants.

In specific embodiments of such compositions the auxiliary agent is selected from the group consisting of liposomes, nanoparticles, carboxymethyl cellulose, hydroxyethyl cellulose, mineral oil, petrolatum, glycerin, polysorbate 80, hydroxyethyl starch, dextran, and polyethylene glycol.

In the inventive compositions the concentration of the active agent usually is in the range of from 10 to 10.000 µM. In some embodiments the lower limit for the concentration of the active agent is 30 µM or even 100 µM. In some embodiments the upper limit is 3.000 µM or 1.000 µM. This results in preferred ranges of e.g. from 30 to 10.000 µM, from 30 to 3.000 µM, from 10 to 3.000 µM, 100 to 3.000 µM and the like.

Referring to the total weight of an inventive composition the concentration of the active agent may vary in specific embodiments within the range of from 1 to 30 wt.-%. In some embodiments the lower limit is 5 wt.-% or even 10 wt.-%. In some embodiments the upper limit is 25 wt.-% or 20 wt.-%. This results in preferred ranges of e.g. from 5 to 30 wt.-%, from 5 to 25 wt.-%, from 10 to 25 wt.-%, 10 to 30 wt.-% and the like.

In specific embodiments such compositions further comprise at least one other active agent being effective in the treatment of skin pigmentation.

In such embodiments the other active agent may be selected from one of the prior art skin pigmentation agents, such as the ones that were mentioned in the introduction.

Generally, the inventive composition may be used in any formulation suitable for treatment of skin pigmentation. In specific embodiments of the invention the composition is formulated in the form of an ointment, a lotion, a cream, a gel, a spray, a plaster or a sustained release plaster. In other specific embodiments of the invention the composition is formulated in the form of a solution. Said solution may be applied by means of a microneedle device or prior to sonication, electrical stimulation, etc.

As mentioned above, the inventive use of the above mentioned active agent may also occur non-therapeutically, wherein non-therapeutically refers to a treatment not being directed to curing, treating or preventing a condition of disorder (see above).

Therefore, the present invention is further directed to a non-therapeutic method of skin pigmentation regulation, wherein an effective amount of at least one of the above mentioned active agent is administered to a subject.

The non-therapeutic method also encloses embodiments, where the above mentioned active agent is administered to the subject to be treated simultaneously, sequentially or separately together with at least one other active agent useful in the treatment of skin pigmentation (see above).

The following examples show some of the features of specific embodiments of the present invention. However, the skilled reader will understand that those embodiments are just exemplary but do not restrict the inventive idea to exactly the features or the combination of features of the embodiments of the examples.

In the description of the examples it is referred to the following figures, wherein
- Figure 1: comprises two microscopic images and a bar graph showing that 2,5-dimethylpyrazine (DMP) increases epidermal melanin content in human skin organ culture,
- Figure 2: comprises two microscopic images and a graph showing that 2,5-dimethylpyrazine (DMP) increases epidermal melanin content in human skin organ culture,
- Figure 3: comprises two microscopic images and a graph showing that 2,5-dimethylpyrazine (DMP) increases expression of the melanosome-melanogenesis marker gp100 in human skin organ culture,
- Figure 4: comprises two microscopic images and a graph showing that 2,5-dimethylpyrazine (DMP) increases tyrosinase activity, a marker of melanogenesis, in human skin organ culture,
- Figure 5: comprises three microscopic images and a graph showing that triphenyl phosphine oxide (TPPO) prevents the effect of 2,5-dimethylpyrazine (DMP) to increase epidermal melanin content in human skin organ culture,
- Figure 6: comprises three microscopic images and a graph showing that triphenyl phosphine oxide (TPPO) prevents the effect of 2,5-dimethylpyrazine (DMP) to increase expression of the melanosome-melanogenesis marker gp100 in human skin organ culture,
- Figure 7: comprises three microscopic images and a graph showing that triphenyl phosphine oxide (TPPO) prevents the effect of 2,5-dimethylpyrazine (DMP) to increase tyrosinase activity, a marker of melanogenesis, in human skin organ culture.

### Examples

### 1. The ex vivo skin organ culture model for assessing skin pigmentation

Samples of human abdominal, temporal, and occipital skin were obtained with written patient consent and under ethical approval from healthy adult human subjects during elective plastic surgery performed for cosmetic reasons. Full-thickness human skin samples were prepared as 4-6 mm punches, placed on filter paper, and cultured at the air-liquid interphase under serum-free conditions in William's E medium supplemented only with insulin, hydrocortisone and L-glutamine, as described previously.

For topical application of 2,5-dimethylpyrazine (DMP) (Sigma), a TRPM5 activator, a stock was prepared in ethanol and dissolved at 37°C at a final concentration of 12.5 mM in a 50% v/v solution of Isopropanol in PBS containing 1g/mL of Polyethylene glycol 6000. For topical application of triphenylphosphine oxid (TPPO) (Sigma), and TRPM5 inhibitor, a stock was prepared in ethanol and dissolved at 37°C at a final concentration of 1.5 mM in a 50% v/v solution of Isopropanol in PBS containing 1g/mL of Polyethylene glycol 6000. Four µL droplets of this viscous solution containing DMP (12.5 mM), TPPO (1.5 mM), their combination, or ethanol as vehicle control were applied daily (up to 3 days) to the epidermis of skin biopsies.

### 2. Activation of TRPM5 with 2,5-dimethylpyrazine (DMP)

Histochemical visualization of melanin in skin sections was performed using the Warthin-Starry Stain Kit (Abcam) or Fontana Masson histochemistry. For Warthin-Starry histochemistry, formalin-fixed human skin/HF frozen sections were rinsed in distilled water and incubated with AgNO3 (0.5% in acidulated water) for 30 seconds at 50°C. Next, the sample was incubated with developer solution (0.42% AgNO₃, 5% Gelatin, and 0.15% hydroquinone in acidulated water) and incubated for 2-5 min at 50°C. The reaction was stopped by consecutive rinses in hot (60°C) tap water for 2 min. Incubation with Na₂S₂O₃ (5% in distilled water) for 1 min, followed by 3 min of consecutive washes in tap water can also be used to remove unbound AgNO3 and to tone the brown coloring of melanin black. Brief counterstain with hematoxylin was used to visualize nuclei. After dehydration, samples were embedded in Eukitt^{®}.

Images were then obtained with a digital microscope (Keyence). Quantitative histomorphometric analysis was performed by assessing the relative intensity and the area of melanin stain in standardized reference areas with ImageJ (NIH; Bethesda, MD, USA). Briefly, for each parameter evaluated, multiple non-consecutive skin sections (at least 280 µm apart from each other) per punch biopsy were stained. Two skin punch biopsies were analyzed per experimental group unless indicated otherwise. The melanin content was then calculated based on the product of the fraction of area occupied by melanin and the mean shade of gray of the melanin, as described in detail elsewhere. Evaluations were performed on multiple different microscopic fields per section. For all parameters, the whole epidermis (or just the basal layer) was used for the measurements. The analysis was performed in the center of the skin biopsy, at least 500 µm away from the edges as these sites show prominent wound-healing processes such as re-epithelialization.

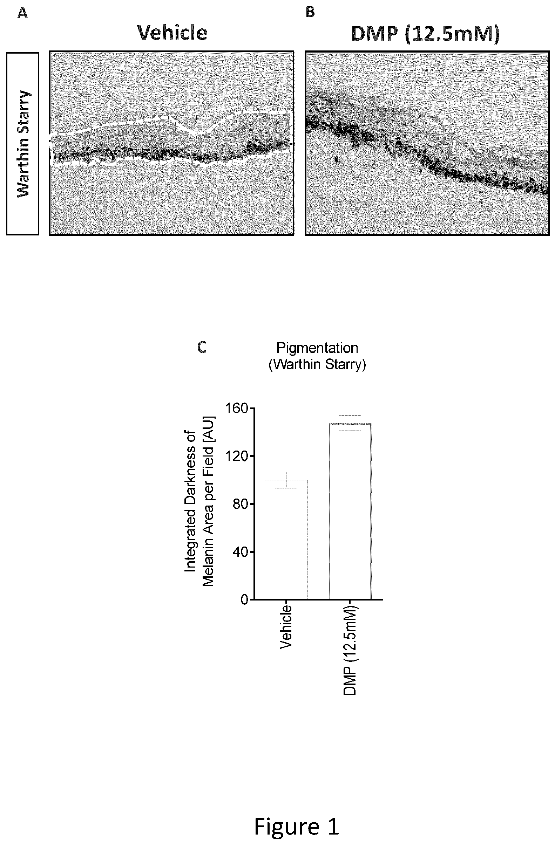

Figure 1 shows representative images (a, b) and the results of the quantitative histomorphometric analysis (c) when performing histochemical visualization of melanin in skin sections using the Warthin-Starry technique. From Figure 1 it can be identified that the topical application of the TRPM5 agonist 2,5-dimethylpyrazine (DMP, 12.5 mM) markedly increases epidermal melanin content after 3 days in human skin organ culture as compared to vehicle. Further, Figure 1 also shows that DMP treatment increased the melanin content not only in the basal layer (i.e. the epidermal compartment where melanocytes reside) but also in the suprabasal layers which suggests an augmented melanosome transfer upon TRPM5 activation by DMP.

For Fontana Masson stain, ethanol/acetic acid-fixed human skin/HF frozen sections were rinsed in two and three washes of Tris-buffered saline and distilled water, respectively. Next, samples were incubated with AgNO3 (5% in distilled water) for 40 min at 56°C in the dark. Samples were rinsed three times with distilled water and then incubated with Na₂S₂O₃ (5% in distilled water) for 1 min at RT. Brief counterstain with hematoxylin was used to visualize nuclei. After dehydration, samples were embedded in Eukitt^{®}. Image acquisition and quantitative histomorphometric analysis were performed similar to as described for the Warthin-Starry technique.

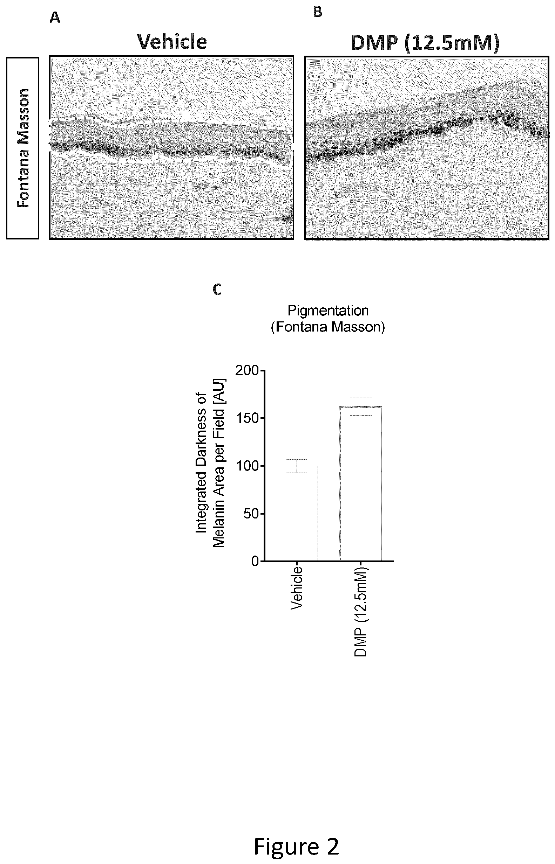

Figures 2 shows representative images (a, b) and the results of the quantitative histomorphometric analysis (c) when performing histochemical visualization of melanin in skin sections using the Fontana-Masson technique, a complementary technique of the Warthin-Starry method. From Figure 2 it can be identified that the topical application of the TRPM5 agonist 2,5-dimethylpyrazine (DMP, 12.5 mM) markedly increases epidermal melanin content after 3 days in human skin organ culture as compared to vehicle, thereby further supporting the pigmentation promoting effect of TRPM5 activation. Further, Figure 2 also shows that DMP treatment increased the melanin content not only in the basal layer (i.e. the epidermal compartment where melanocytes reside) but also in the suprabasal layers which suggests an augmented melanosome transfer upon TRPM5 activation by DMP.

For the detection of gp100 (which is known to be involved in melanosome-trafficking compartment, contributes to the functional integrity and assembly of melanosomes, and hence participates in *de novo* melanin synthesis), acetone-fixed, human skin/HF frozen sections were incubated overnight at 4°C with anti-gp100 (clone NKI/beteb; MONO-SAN) diluted 1:100 with Antibody Diluent (Agilent Dako). After consecutive rinses in PBS, samples were incubated with rhodamine-conjugated secondary antibody (Jackson ImmunoResearch) diluted 1:200 in normal goat serum (2% in PBS) for 45 min at RT. After rinsing with PBS, samples were counterstained with DAPI and mounted on glass slides with Fluoromount-G^{®} (SouthernBiotech). Image acquisition and quantitative histomorphometric analysis were performed similar to as described for the Warthin-Starry technique, with a special emphasis on assessing the relative intensity and the area of gp100-specific immunoreactivity (IR) in the basal epidermal layer (i.e. the epidermal compartment where melanocytes reside).

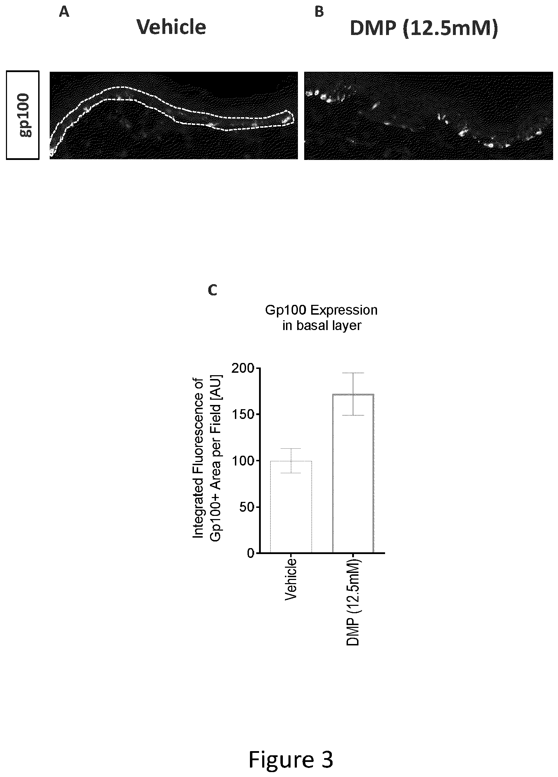

Figures 3 shows representative images (a, b) and the results of the quantitative histomorphometric analysis (c) when performing immunohistochemical visualization of gp100-specific IR in skin sections. From Figure 3 it can be identified that the topical application of the TRPM5 agonist 2,5-dimethylpyrazine (DMP, 12.5 mM) markedly increases the intensity level of gp100-specific IR in the basal epidermal layers after 3 days in human skin organ culture as compared to vehicle, thereby further supporting the pigmentation promoting effect of TRPM5 activation.

For the *in situ* detection of tyrosinase activity (the enzyme that catalyzes the rate-limiting step of melanin synthesis), human skin cryosections were fixed in an acetone/methanol solution (1:1 ratio) and blocked with H₂O₂ (0.3% in PBS) for 15 min. Endogenous biotin was blocked using Streptavidin/Biotin Blocking Kit (Vector Labs). Then, samples were blocked with normal goat serum (5%) and BSA (1%) in PBS for 30 min. Tyrosinase activity was detected with the TSA^{™} Biotin System (Akoya Biosciences).

In brief, samples were incubated with Biotin Tyramide (1:50 in Amplification Diluent) and then consecutively rinsed with IGEPAL^{®} CA-630 (Sigma, 0.1% in PBS) and PBS. For visualization of biotin cross-linked in the vicinity of tyrosinase activity, samples were incubated with Strepdavidin-Cy3 (Sigma, 1:600 in 5% BSA in PBS) for 1 hour. Following additional rinses with GEPAL^{®} CA-630 (0.1% in PBS) and PBS, samples were counterstained with DAPI and mounted on glass slides with Fluoromount-G^{®} (SouthernBiotech).

Image acquisition and quantitative histomorphometric analysis were performed similar to as described for the Warthin-Starry technique, with a special emphasis on assessing the relative intensity and the area of tyrosinase activity-specific immunoreactivity (IR) in the basal epidermal layer (i.e. the epidermal compartment where melanocytes reside).

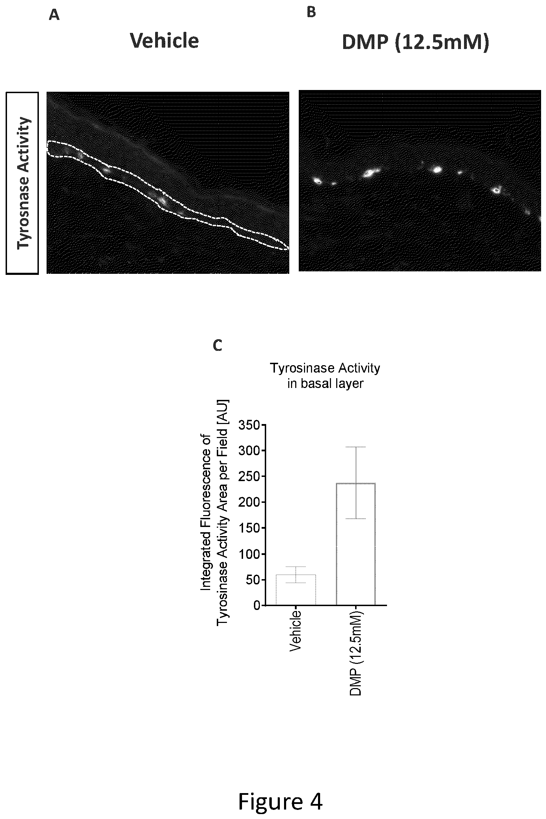

Figures 4 shows representative images (a, b) and the results of the quantitative histomorphometric analysis (c) when performing histochemical visualization of tyrosinase activity in skin sections. From Figure 4 it can be identified that the topical application of the TRPM5 agonist 2,5-dimethylpyrazine (DMP, 12.5 mM) markedly increases the intensity level of tyrosinase activity in the basal epidermal layers after 3 days in human skin organ culture as compared to vehicle, thereby further supporting the pigmentation promoting effect of TRPM5 activation.

### 3. Inactivation of TRPM5 with Triphenyl phosphine oxide (TPPO)

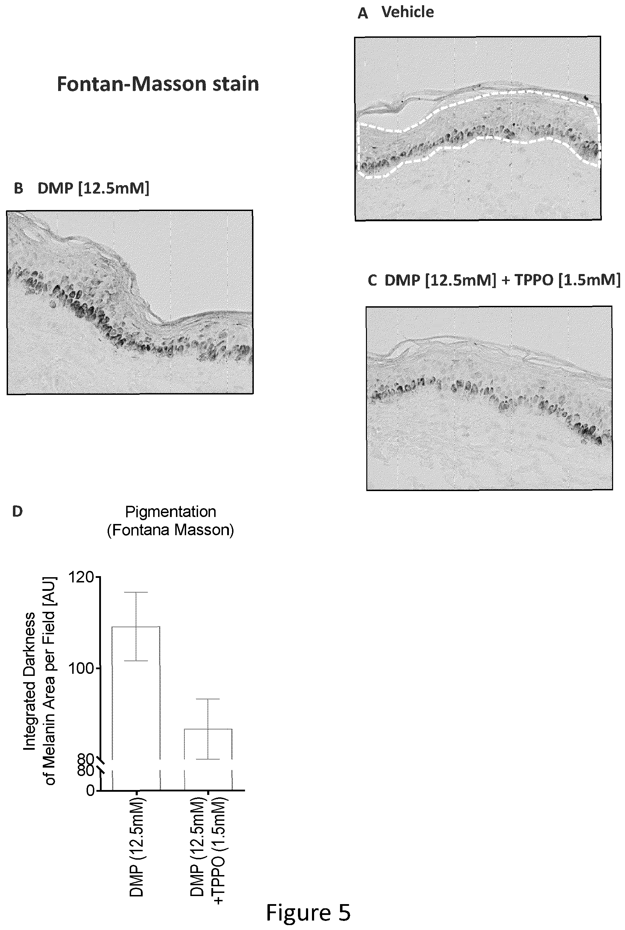

Figures 5 shows representative images (a, b, c) and the results of the quantitative histomorphometric analysis (d) when performing histochemical visualization of melanin in skin sections using the Fontana-Masson technique. From Figure 5 it can be identified that the TRPM5 antagonist triphenyl phosphine oxide (TPPO, 1.5 mM) prevented the effect of the TRPM5 agonist 2,5-dimethylpyrazine (DMP, 12.5 mM) to increase epidermal melanin content after 3 days in human skin organ culture as compared to vehicle. This suggests that the pigmentation-promoting effect of DMP is mediated by TRPM5.

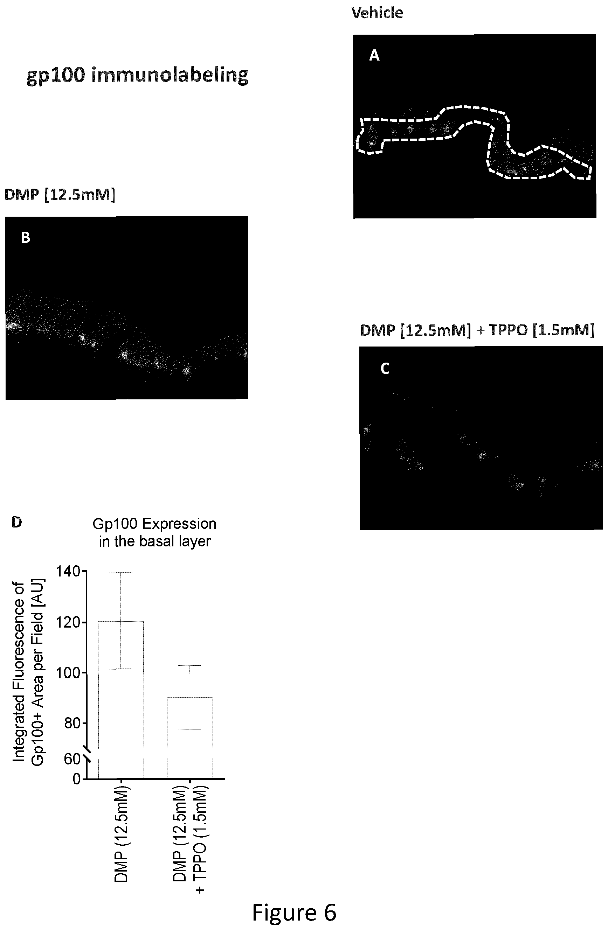

Figures 6 shows representative images (a, b, c) and the results of the quantitative histomorphometric analysis (d) when performing immunohistochemical visualization of gp100-specific IR in skin sections. From Figure 6 it can be identified that the TRPM5 antagonist triphenyl phosphine oxide (TPPO, 1.5 mM) prevented the effect of the TRPM5 agonist 2,5-dimethylpyrazine (DMP, 12.5 mM) to increase gp100-specific IR in the basal epidermal layers after 3 days in human skin organ culture as compared to vehicle. This suggests that the pigmentation-promoting effect of DMP is mediated by TRPM5.

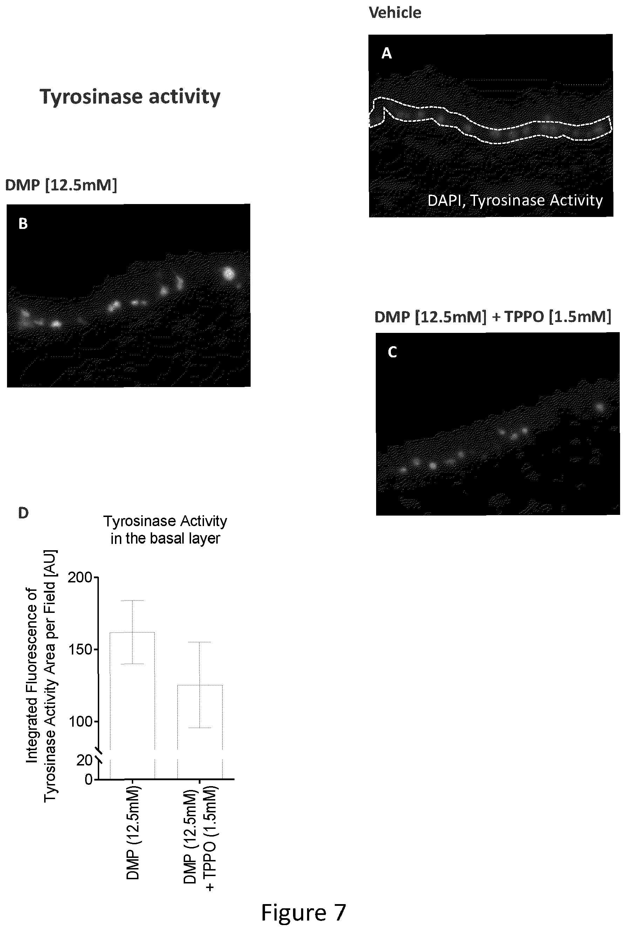

Figures 7 shows representative images (a, b, c) and the results of the quantitative histomorphometric analysis (d) when performing histochemical visualization of tyrosinase activity in skin sections. From Figure 7 it can be identified that the TRPM5 antagonist triphenyl phosphine oxide (TPPO, 1.5 mM) prevented the effect of the TRPM5 agonist 2,5-dimethylpyrazine (DMP, 12.5 mM) to increase tyrosinase activity in the basal epidermal layers after 3 days in human skin organ culture as compared to vehicle. This suggests that the pigmentation-promoting effect of DMP is mediated by TRPM5.

## Claims

1. Active agent for use in the treatment of skin pigmentation, wherein said active agent activates, enhances, inactivates, blocks or dampens the cellular response of the transient receptor potential ion channel TRPM5 or interferes with the expression of said ion channel.

2. Active agent for use in the treatment of skin pigmentation according to claim 1, wherein the active agent is an agonist of the transient receptor potential ion channel TRPM5 for use in the treatment of hypopigmentation.

3. Active agent for use in the treatment of skin pigmentation according to claim 1, wherein the active agent is an antagonists/inverse agonists of the transient receptor potential ion channel TRPM5 for use in the treatment of hyperpigmentation.

4. Active agent for use in the treatment of skin pigmentation according to one of the claims 1 to 3, wherein said active agent is selected from
a) any one of the TRPM5 activating agonists dimethylpyrazine, dimethylethylpyrazine, 2-heptanone, SID2848719 (CAS number 702636-90-6), rutamarin, xanthotoxin, isopimpinellin, carbachol, 3-deoxyglucosone, (E)-N-(3,4dimethoxybenzylidene)-2-naphthalene-1-yl) acetohydrazide or a combination thereof, or an aptamer binding to TRPM5 and activating or enhancing said ion channel to produce a cellular response, or
b) any one of the TRPM5 inactivating antagonists/inverse agonists triphenylphosphine oxide, econazole, miconazole, chlorpromazine or a combination thereof, or an aptamer binding to TRPM5 and inactivating, blocking or dampening said ion channel to produce a cellular response, or
c) the active agent is selected from miRNA, siRNA or a ribozyme targeted to the TRPM5 gene or targeted to the mRNA corresponding to the TRPM5 gene.

5. Active agent for use in the treatment of skin pigmentation according to one of the claims 1 to 4, wherein the treatment is
a) for treating a hyperpigmentation disorder being selected from lentigines, solar lentigo, melasma, chloasma, ephelides, hyperpigmented birthmarks such as nevus of Ota, Mongolian spots, cafe-au-lait spots, pigmented nevi (moles), post-inflammatory hyperpigmentation, post-infection hyperpigmentation, post-blister hyperpigmentation, post-burn hyperpigmentation, post-traumatic hyperpigmentation, post-birth hyperpigmented stretch marks [striae distensae]), or
b) for treating a hypopigmentation disorder being selected from vitiligo, post-inflammatory hypopigmentation, post-infection hypopigmentation, post-blister hypopigmentation, post-burn hypopigmentation, post-traumatic hypopigmentation, post-birth hypopigmented stretch marks [striae distensae], post-bleaching hypopigmentation.

6. Use of an active agent according to one of the claims 1 to 5 as a cosmetic for the non-therapeutical treatment of skin pigmentation.

7. Cosmetic or medical skin pigmentation composition comprising at least one active agent according to one of the claims 1 to 4 and at least one auxiliary agent selected from the group consisting of carriers, excipients, adjuvants, diluents, and disintegrants.

8. Cosmetic or medical skin pigmentation composition for according to claim 7, wherein the auxiliary agent is selected from the group consisting of liposomes, nanoparticles, carboxymethyl cellulose, hydroxyethyl cellulose, mineral oil, petrolatum, glycerin, polysorbate 80, hydroxyethyl starch, dextran, and polyethylene glycol.

9. Cosmetic or medical skin pigmentation composition according to claim 7 or claim 8, further comprising at least one other active agent being effective in the treatment of skin pigmentation.

10. Cosmetic or medical skin pigmentation composition according to one of the claims 7 to 9, wherein the composition is formulated in the form of an ointment, a lotion, a cream, a gel, a solution, a spray, a plaster or a sustained release plaster.

11. Non-therapeutic method of skin pigmentation regulation, wherein an effective amount of at least one active agent that activates, enhances, inactivates, blocks or dampens the cellular response of the transient receptor potential ion channel TRPM5 or interferes with the expression of said ion channel is administered to a subject.
